# EUROPÄISCHE PATENTSCHRIFT

(11) **EP 3 288 596 B1**
(45) Veröffentlichungstag und Bekanntmachung des Hinweises auf die Patenterteilung: **20.03.2019**
(21) Anmeldenummer: 16718357.3
(22) Anmeldetag: 25.04.2016
(51) Int. Cl.: A61L 2/00, B01J 19/00, A61L 2/04, A61L 2/10

(54) **VERFAHREN ZUR KONTINUIERLICHEN VIRUSINAKTIVIERUNG IN EINEM MIKROREAKTOR**
METHOD FOR CONTINUOUS VIRAL INACTIVATION IN A MICROREACTOR
PROCÉDÉ D'INACTIVATION EN CONTINU DE VIRUS DANS UN MICROREACTEUR

(30) Priorität: 28.04.2015 EP 15165505
(43) Veröffentlichungstag der Anmeldung: 07.03.2018
(73) Patentinhaber: Bayer Aktiengesellschaft, 51373 Leverkusen (DE)
(72) Erfinder: SCHWAN, Peter, 51373 Leverkusen (DE); VESTER, Andrea, 40591 Düsseldorf (DE); LOBEDANN, Martin, 51069 Köln (DE)
(74) Vertreter: BIP Patents
(86) Internationale Anmeldenummer: PCT/EP2016/059169
(87) Internationale Veröffentlichungsnummer: WO 2016/173982

(56) Entgegenhaltungen:
- WO-A1-2006/039568
- WO-A2-2004/103539
- US-A1- 2009 269 250

## Beschreibung

Die vorliegende Erfindung betrifft ein Verfahren zur kontinuierlichen Virusinaktivierung in einer Verweilzeitstrecke.

Biopharmazeutische Produktionsprozesse erfordern verschiedene orthogonale Schritte zur Virenreduktion. Ein häufig eingesetztes Verfahren zur Inaktivierung von (umhüllten) Viren ist der Kontakt mit einem sauren Medium.

Virusinaktivierung bei niedrigem pH-Wert im Batch-Modus ist in der biopharmazeutischen Produktion von Wirkstoffen, z. B. Antikörpern, bekannt und vielfach angewendet (Sofer 2003, Virus Inactivation in the 1990s - and into the 21st Century. Part 4. BioPharm International). Hierbei wird das zu inaktivierende Gut, eine Flüssigkeit, die aktive Viren potentiell enthält, in einen geeigneten Behälter eingebracht, mit Hilfe einer sauren Lösung auf einen pH-Wert ≤ 4 eingestellt, wenn nötig homogenisiert, und über die geforderte Zeit ruhen gelassen. Die Inaktivierung der Viren geschieht durch den Kontakt der Viren mit der sauren Lösung über eine bestimmte produkt- und prozessabhängige Zeit. Der gesamte Inhalt des Bags erfährt somit die Inaktivierung mit nahezu identischer Verweilzeit und folglich ist die geleistete Virenreduktion in jedem Fluidelement des Behälters ebenfalls nahezu gleich.

Soll nun ein Prozess zur Produktion biopharmazeutischer und biologischer Produkte insbesondere pharmazeutischer Antikörper im kontinuierlichen Betriebsmodus gefahren werden, so müsste die benötigte Haltezeit (=Verweilzeit) für die Virusinaktivierung realisiert werden.

Eine kontinuierliche Virusinaktivierung bedeutet im Sinne der Anmeldung, dass die Zufuhr von Feedstrom in das Virusinaktivierungsmodul und die Abfuhr des Produktstroms aus dem Virusinaktivierungsmodul pausenlos erfolgen.

Eine Möglichkeit eine kontinuierliche Virusinaktivierung durchzuführen, ist die Bestrahlung mit UV-C Licht. WO2002038191, EP1339643B1, EP1464342B1, EP1914202A1 und EP1916224A1 beschreiben die Verwendung einer helixförmigen Verweilschleife in der das zu inaktivierende Gut mit UV-C Licht bestrahlt und die vorhandenen Viren in der Folge inaktiviert werden. Durchströmt ein Fluid ein helikal gewickeltes Rohr, so wirkt die Zentrifugalkraft auf das Fluid. Durch diese Zentrifugalkräfte werden Sekundärströmungen (sog. Dean-Wirbel) induziert, was zu einer verbesserten radialen Durchmischung und somit homogeneren Bestrahlung des zu inaktivierenden Guts führt. Die in den genannten Quellen verwendete Helixstruktur ist eine gerade Helixwicklung ohne Richtungsänderungen der Achse der Helix. Für die Anwendung einer kontinuierlichen Virusinaktivierung bei niedrigem pH-Wert ist die Verwendung einer geraden Helix-Struktur, wie sie bei der UV-C Bestrahlung verwendet wird, nicht praktikabel, da die Verweilzeitverteilung zwar enger als beim laminar durchströmten geraden Rohr ist, jedoch immer noch zu breit ausfällt. Bedingt durch die immer noch vergleichsweise breite Verweilzeitverteilung würde auch diese Geometrie für eine pH-Virusinaktivierung weiterhin eine große Anlage erfordern.

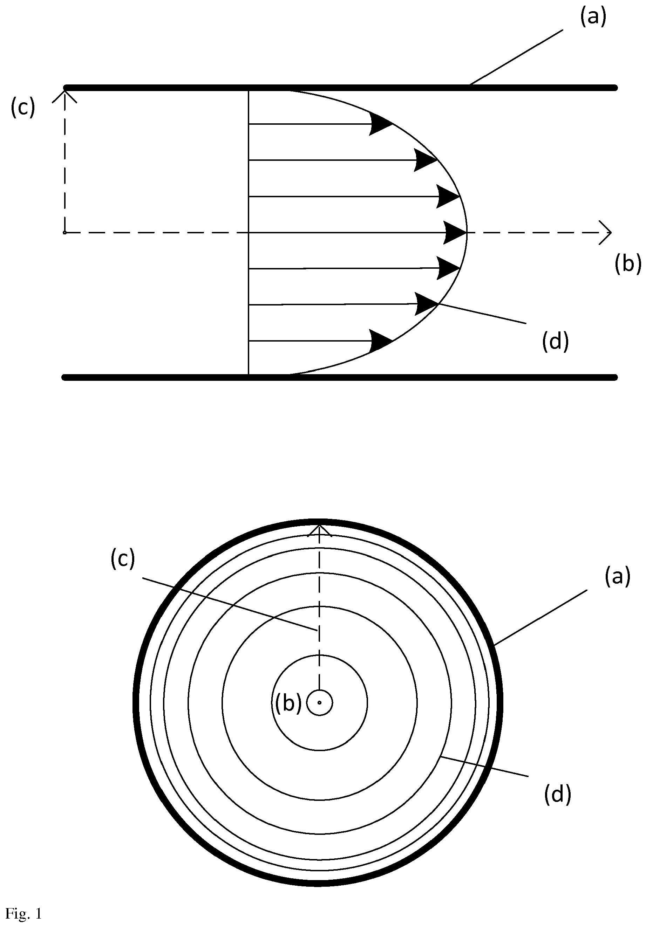

In einer laminaren Rohrströmung bildet sich ein parabolisches Geschwindigkeitsprofil aus, wodurch es zu einer breiten Verweilzeitverteilung kommt (**Fig. 1**). Da die Maximalgeschwindigkeit im Zentrum der Rohrströmung das Doppelte der mittleren Geschwindigkeit beträgt, an den Rohrwänden jedoch die Geschwindigkeit gleich Null ist (Haftbedingung), tritt in diesen Fällen eine sehr breite Verweilzeitverteilung auf. Die so erhaltenen Verweilzeiten liegen zwischen der halben durchschnittlichen Verweilzeit (hervorgerufen durch die schnell strömenden Fluidelemente in der Rohrmitte) und einer unendlich langen Verweilzeit (hervorgerufen durch die anhaftenden Fluidelemente in Wandnähe). Da zum einen zur effektiven Inaktivierung der Viren eine Mindestverweilzeit erforderlich ist, zum anderen aber lange Verweilzeiten bei niedrigem pH-Wert das Produkt (wie z. B. ein Protein) schädigen könnte, ist die Realisierung einer engen Verweilzeitverteilung im kontinuierlichen Betrieb unabdingbar. Ein Wechsel von der laminaren Strömungssituation in eine turbulente Pfropfenströmung mit einheitlicher Verweilzeit stellt in diesem Fall keine akzeptable Altenative dar. Turbulente Strömungen setzen hohe Fließgeschwindigkeiten voraus. Sollen nun die für Virusinaktivierungen bei niedrigem pH-Wert üblichen langen Verweilzeiten (beispielsweise 60-120 min) realisiert werden, entstehen unvorteilhaft große Anlagen, welche auch einen großen Druckverlust aufweisen.

WO1998/02237 beschreibt eine Lösung des Problems des parabolischen Geschwindigkeitsprofils in einem kontinuierlich betriebenen Rohrreaktor zur Fällung von Produkten aus einer flüssigen Reaktionsmischung durch die Anwendung von segmentierter Fahrweise (Segmented Flow Processes), in dem diskrete Volumen der Reaktionsmischung von diskreten Volumen einer mit der Reaktionsmischung nicht mischbarem Trennflüssigkeit getrennt sind, wobei die Verweilzeit der Reaktionsmischung im Rohrreaktor für die Fällung ausreichend ist. Die diskreten Volumen werden unter Propfenströmungsbedingungen, - so genannte "Plug Flow" Bedingungen - erzeugt und für jedes Volumen sind die Reaktionsbedingungen im Wesentlichen identisch, so dass ein uniformes Produkt für jedes Volumen erhalten wird.

Tuercke et al. beschreiben die segmentierte Führung - Flüssigkeit/Flüssigkeit oder Flüssigkeit/Gas - in mikrostrukturierten Reaktoren im kontinuierlichen Betrieb für die organische Synthese und Herstellung mikroskaliger partikulärer Produkte wie z. B. multiple Emulsionen und Nanopartikeln und Polymerisierung (Organic Process Research & Development 2009, 13, 1007-1013). Die Methode der segmentierten Phase wurde auch vom Fraunhofer-Institut für Chemische Technologie ICT zur Trennung von Zellen verwendet. DieTechnik der segmentierten Phasen wird außerdem bei der Probenförderung im Baychromat® System zur Probenahme und Analyse eingesetzt (US 2009/0178495).

Die Anwendbarkeit der Propfenströmungsbedingungen oder segmentierten Strömung für Verfahren, die gleichzeitig eine lange Verweilzeit und eine enge Verweilzeitverteilung erfordern, wie z. B. die Virusinaktivierung bei niedrigen pH-Wert, wurde bisher nicht untersucht oder erwähnt.

Ausgehend aus dem Stand der Technik bestand die Aufgabe darin, eine neue, einfache und preiswerte Lösung zur Verfügung zu stellen, welche die benötigte Verweilzeit in einer kontinuierlich durchströmten Verweilzeitstrecke zur kontinuierlichen Virusinaktivierung insbesondere bei niedrigem pH-Wert bei enger Verweilzeitverteilung ermöglicht.

Die Erfindung löst diese Aufgabe durch ein Verfahren zur kontinuierlichen Virusinaktivierung eines zu inaktivierenden Produktstroms in einem Reaktor 1 mit einem geringen hydraulischen Durchmesser von 0,01 mm bis 6 mm, bevorzugt 0,5 mm bis 3 mm, umfassend folgende Schritte:
a) Bereitstellen des zu inaktivierenden Produktstroms,
b) Einstellung der vireninaktivierenden Bedingungen,
c) Einbringen in den Produktstrom eines mit dem Produktstrom nicht mischbaren Trennmediums zur Segmentierung des Produktstroms,
d) Zufuhr und Durchlaufen des segmentierten Produktstroms aus c) unter vireninaktivierenden Bedingungen in einer Verweilstrecke, gebildet durch den Reaktor 1
e) Ausströmen aus der Verweilstrecke,
f) Bevorzugt kontinuierliche Abtrennung des Trennmediums.

Vorzugsweise ist der Reaktor sowie die Elemente des Moduls zur Segmentierung des Produktstroms, die mit dem Produktstrom in Kontakt treten, sterilisierbar, bevorzugt autoklavierbar , gamma-bestrahlbar oder mit Ethylenoxid (ETO) begasbar, was einen keimarmen oder sogar sterilen Betrieb ermöglicht.

Bevorzugt ist der Reaktor ein Rohreaktor. Besonders bevorzugt wird ein Rohrreaktor aus einem Einwegmaterial, z.B. ein Schlauch, verwendet, welcher nach Gebrauch verworfen wird um auf die Reinigung verzichten zu können. Für diese Eigenschaft wird bevorzugt ein Schlauch verwendet, der den einschlägigien Qualitätsansprüchen, z. B. medizinische Qualität (USP Class VI) entspricht. Beispielsweise ist der Rohrreaktor ein Schlauch aus Silikon. Als Beispiel werden die Schläuche Pharmed®-BPT (Silikonschlauch), C-Flex-374® (thermoplastischer Schlauch), oder Sanipure® der Firma Saint-Gobain Performance Plastics genannt ohne sich darauf zu begrenzen. In der Testanlage wurden ein kommerzieller Schlauch aus SaniPure® mit einem Innendurchmesser von 1,6 mm verwendet.

Die geometrische Gestaltung des Rohrreaktors in seiner Länge ist beliebig: gerade, gewickelt oder gekrümmt, vorausgesetzt er knickt nicht ab. Bevorzugt wird eine platzsparende Anordnung des Rohrreaktors. Typischerweise wird der Rohrreaktor von einer Trägerstruktur getragen. Z. B. wird der Rohrreaktor um übereinander auf einem Gestell befestigte Rahmen gewickelt, wobei die Rahmen rund oder eckig sein können. Auch eine helikale Wicklung um eine oder mehrere Säule herum ist möglich. Für eine UV- Inaktivierung kann die Säule dann eine UV-Lampe aufweisen und das Rohrreaktor UVdurchlässig sein. Auch können thermische virusinaktivierenden Bedingungen durch Erhitzen der Trägerstruktur im Rohrreaktor eingestellt werden. Für eine thermische Inaktivierung können die umwickelten Strukturen auch in ein Flüssigkeitsbad eingebracht werden, um somit scharfe Temperaturänderungen herbeizuführen.

Alternativ kann ein Rohreaktor verwendet werden, der durch eine oder mehrere aufeinander gestapelten Platten, insbesondere Kunststoffplatten, in denen ein Kanal mit einem Einlass und einen Auslass eingearbeitet ist, gebildet ist. Umfasst dieser Plattenreaktor mehrere Platten, sind der Einlass und Auslass der mittleren Platten so positioniert, dass durch das Stapeln ein durchgängiger Kanal mit der gewünschten Länge gebildet wird. Auch die geometrische Gestaltung des Kanals in seiner Länge ist beliebig: gerade, gewickelt oder gekrümmt.

Der Querschnitt des Reaktors 1 ist typischerweise rund oder oval, kann aber auch eckig sein.

Im Schritt a) wird ein Produktstrom an Flüssigkeit bereitgestellt, der sowohl Produkt als auch potentiell zu inaktivierende Viren enthalten kann.

Als mögliche vireninaktivierenden Bedingungen für Schritt b) werden ein niedriger pH (bevorzugt ≤ 4), Detergenzien, UV- oder thermische Behandlung genannt.

Bevorzugt wird im Schritt b) der pH-Wert des Produktstroms auf einen Wert ≤ 4 eingestellt, sofern der pH-Wert des zu inaktivierenden Guts nicht bereits den geforderten Wert hat. Der pH-Wert des Produktstroms wird üblicherweise vor Eintritt in die Vorrichtung zur Virusinaktivierung durch einen Sensor erfasst (**Fig. 8**). Üblicherweise hat dieser pH-Sensor keine Regelaufgaben. Das Aufzeichnen des pH-Signals dient lediglich zur Prozessüberwachung. Einstellen des pH-Wertes der zu inaktivierenden Lösung auf ≤ 4 kann beispielsweise durch Zugabe von HCl-Lösung erfolgen. Die Zugabe erfolgt typischerweise im Vorfeld der Vorrichtung zur Virusinaktivierung. Nach Schritt e) oder f) wird üblicherweise der pH-Wert auf >4 mit einer Base beispielsweise NatriumhydroxidLösung (NaOH) eingestellt, um die Virusinaktivierung zu beenden. Die Neutralisierung kann als Batch-Operation oder als kontinuierliches Produktionsverfahren durchgeführt werden und somit in einen Batch-Prozess oder in einen kontinuierlichen Prozess integriert werden.

Als Trennmittel wird bei dem erfindungsgemäßen Verfahren eine mit dem Produktstrom nichtmischbare Phase verwendet. Bevorzugt ist das Trennmittel ein Öl oder ein Gas wie zum Beispiel Luft, CO₂ oder Stickstoff, bevorzugt ein Gas, besonders bevorzugt Stickstoff, aufgrund seiner Reaktionsträgheit gegenüber dem Produktstrom und niedrige Löslichkeit im wässrigen Produktstrom.

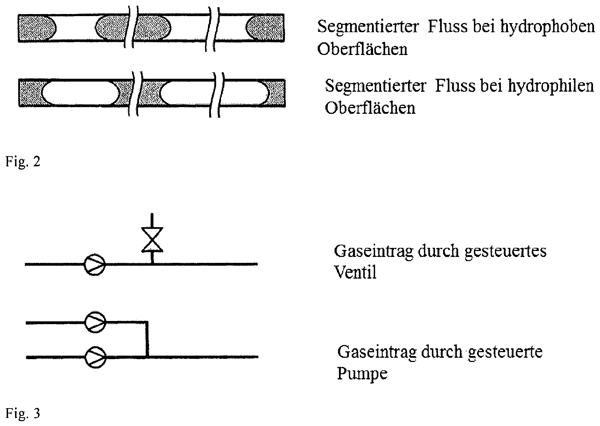

Für das Einbringen des Trennmittels und Segmentierung des Produktstroms im Schritt c) weist der Reaktor typischerweise zusätzlich zu einem Eintritt 4 einen Eintritt 6 für das Trennmittel üblicherweise in Form eines T-Stücks auf, an dem ein Mittel zur Pulseinführung des Trennmittels - entweder ein angesteuertes, sich öffnendes Ventil mit angeschlossener Druckleitung oder eine Pumpe - angeschlossen ist (Fig. 3). Die Segmentierung erfolgt beispielsweise mit einer Pumpe mit einer Pulsation von 0,1 bis 200 pro Minute.
Üblicherweise wird der Reaktorstrom mit einem Volumenstrom von 1 bis 1000 L/min, bevorzugt 10 bis 100 mL/min durchströmt.

Alternativ zur gepulsten Einführung kann das Trennmittel über eine Membran kontinuierlich zugeführt werden. In dieser Ausführungsform wird ein Modul zur Segmentierung des Produktstroms verwendet, das eine oder mehrere Hohlfasern mit einer hydrophoben Wand umfasst, durch die das Trennmittel in den Produktstrom eingeführt wird. Möglich ist auch die Anwendung eines Hohlfasermoduls mit einer hydrophilen Wand, wobei im Lumen der Hohlfasern das Trennmittel kontinuierlich befördert wird und durch die Wand der Produkstrom eingeführt wird. Diese zweite Ausführungsform setzt voraus, dass die Poren der Hohlfasen das Produkt durchlassen. Die Anwendung einer Membransegmentierung setzt im allgemein voraus, dass die vireninaktivierenden Bedingung die nötigen Eigenschaften der Membran nicht beeinträchtigen. Bei der Nutzung von Detergenzien wird die Anwendung eines T-Stücks daher bevorzugt.

Eine chemische Einbringung eines Trennmittels z. B. von CO₂ wäre auch möglich, insbesondere wenn die vireninaktivierenden Bedingung pH-Variationen tolerieren.

Durch solche Segmentierungen des Produktstroms werden üblicherweise Produktstromvolumen von 0,1 ml bis 100 ml gebildet mit Volumenabstände von 0,1 ml bis 10 ml zwischen zwei Produktstromvolumen.

Üblicherweise beträgt die Mindestlänge eines Segments insbesondere eines Trennmittelsegments - drei mal das Innendurchmesser des Reaktors. Eine sinnvolle maximale Länge eines Segments ist ein Fünftel der Verweilstrecke.

Aufgrund der Kapillarwirkung und der Oberflächenspannung im Reaktor wird die Segmentierung der Phasen erhalten, sodass zwei Segmente einer Phase durch ein Segment der anderen Phase getrennt sind. Dadurch wird eine Rückvermischung zwischen zwei Segmenten einer Phase minimiert und die Verweilzeitverteilung des Gesamtsystems wird enorm eingeengt.

Die einzeln transportierten Produktstromsegmente (=Produktstromvolumen) können als kleine Inaktivierungsbehältnisse aufgefasst werden, welche immer vollständig geleert werden und sich untereinander auch nur minimal vermischen.

Üblicherweise wird der Produktstrom im Schritt d) mit einer Strömungsgeschwindigkeit von 0,1 bis 1000, bevorzugt 1 bis 100, besonders bevorzugt 10 bis 100 ml/min üblicherweise mit Hilfe einer Pumpe dem Reaktor zugeführt und befördert. In diesem Schritt erfolgt die gewünschte Kontaktzeit (=Verweilzeit) zwischen den vireninaktivierenden Bedingungen, insbesondere der saurer Lösung, und eventuell vorhandenen Viren. Die Verweilzeit ist lang genug, um die Viren zu inaktivieren, ohne das Produkt zu sehr zu schädigen. Sie wird vor der Umsetzung in einem kontinuierlichen Verfahren üblicherweise experimentell in einem Batch-Verfahren ermittelt und beträgt typischerweise von 30 min für pH-empfindliche Produkte bis 10 h für weniger empfindliche Produkte. Die erforderliche Verweilzeit sowie die Maximalverweilzeit sind produktabhängig. Die Maximalverweilzeit wird üblicherweise so optimiert, dass das Produkt minimal beschädigt wird, um den Bedarf an nachgeschalteten Reinigungschritten so gering wie möglich zu halten.

Als Designparameter für das erfindungsgemäße Verfahren sind entsprechend zu nennen:
- Rohrinnendurchmesser dᵢ des Reaktors
- Rohrlänge L, wobei die Rohrlänge L und innerer Durchmesser des Rohrs an die Dimensionen der Gesamtanlage/Durchflussrate der Anlage so angepasst werden, dass die im jeweiligen Anwendungsfall geforderten Verweilzeiten eingehalten werden.
- Gewünschter Volumenstrom, Produktstromvolumen, Trennmittelvolumen und Pulsrate.

Die kontinuierliche Abtrennung des Trennmittels erfolgt üblicherweise durch einen Abscheider, welcher über Schwerkraft, Zentrifugalkraft oder durch Membraneigenschaften wirkt.
Wird ein Gas als Trennmittel verwendet, wird der Volumenstrom üblicherweise kontinuierlich entgast. Hierfür kann eine Blasenfalle, ein Entlüftungsventil oder bevorzugt ein Membranentgasungsmodul verwendet werden.

Erfordert der Produktionsprozess eine oder mehrere Einstellungen des pH-Wertes wird die Vorrichtung zur Virusinaktivierung üblicherweise an eine Unit zur Einstellung des pH-Wertes angeschlossen. Üblicherweise werden zwei Units zur Einstellung des pH-Wertes verwendet, die erste vor der Inaktivierung zur Einstellung des Produktstroms auf einen pH-Wert ≤ 4, eine weitere nach der Inaktivierung zur Neutralisierung des Produktstroms.

Wird die Vorrichtung zur Virusinaktivierung in einen kontinuierlichen Produktionsprozess integriert, so wird eine oder mehrere Units zur Einstellung des pH-Wertes bevorzugt, in der der Produktstrom eine Rezirkulationsschleife durchströmt. **Fig. 8** stellt die Virusinaktivierung und eine anschließende Neutralisierung exemplarisch dar, ohne sich darauf zu begrenzen. M0503 fördert den Produktstrom in den Bag B0502, wo der pH-Wert nach Verlassen der Virusinaktivierung auf einen Wert ≥ 4 eingestellt wird. Den Inhalt des Bags B0502 fördert die Rezirkulationspumpe M0504 durch die Rezirkulationsschleife in der der pH-Sensor pH0502 den pH-Wert des Produktstroms misst. Strömungstechnisch hinter dem Sensor pH0502 wird das Stellmittel zur Anpassung des pH-Werts zudosiert um den pH-Wert zu regeln. Dies erfolgt über die Vorgabe der Drehzahl für M0505.

In dem erfindungsgemäßen Verfahren ist der zu inaktivierende Produktstrom üblicherweise eine Lösung aus einem Bioreaktor oder einer Chromatographiesäule, insbesondere eine Protein- oder Peptidlösung wie z. B. eine Antikörperlösung.

Der technische Vorteil der erfindungsgemäßen kontinuierlichen Virusinaktivierung gegenüber der im Stand der Technik üblichen Virusinaktivierung im Batch-Modus besteht in der Integrierbarkeit in einen kontinuierlichen Aufarbeitungsprozess, auch "Downstream Processing" genannt, ohne die Prozessfahrweise ändern zu müssen. Hierbei kommt es nicht zu einem Wechsel in der Prozessführung von Batch auf kontinuierlich und wieder zurück, sondern der gesamte "Downstream Processing" ggf. der gesamte Produktionsprozess (Upstream und Downstream) kann kontinuierlich durchfahren werden. Auch kann eine kontinuierliche Virusinaktivierung leichter mit einem kontinuierlichen Teilschrittes eines sonst batchweisen Aufarbeitungsprozesses kombiniert werden.

Die vorliegende Erfindung einschließlich bevorzugter Ausführungsformen wird in Verbindung mit den nachfolgenden Zeichnungen und Beispielen erläutert, ohne hierauf beschränkt zu sein. Die Ausführungsformen können beliebig miteinander kombiniert werden, sofern sich nicht eindeutig das Gegenteil aus dem Kontext ergibt.

Die verwendeten Bezugszeichen sind:
1 = gekrümmte und/oder helixförmig gewickeltes Rohr oder Schlauch
2 = Richtungsumkehrungen und/oder Knicke 2 der Windungsachse h mit einem Winkel α von 45° bis 180°
3 = Rahmen
4 = Eintritt
5 = Austritt
6 = Haltegestell
7 = Fuß
8 = Produktstromleitung

**Fig. 1** zeigt ein Parabolisches Strömungsprofil des laminar durchströmten Rohres (oben: Längsschnitt des Rohres). Linien gleicher Geschwindigkeit in Strömungsrichtung im laminar durchströmten Rohr (unten: Querschnitt des Rohres).
   a = Rohrwand
   b = Axialrichtung des Rohres in Strömungsrichtung
   c = Radialrichtung
   d = Linien gleicher Strömungsgeschwindigkeit in Strömungsrichtung
**Fig. 2** zeigt das Prinzip der Segmentierung
**Fig. 3** zeigt alternative Mittel zur Pulseinführung des Trennmittels angebunden an den Rohrreaktor.

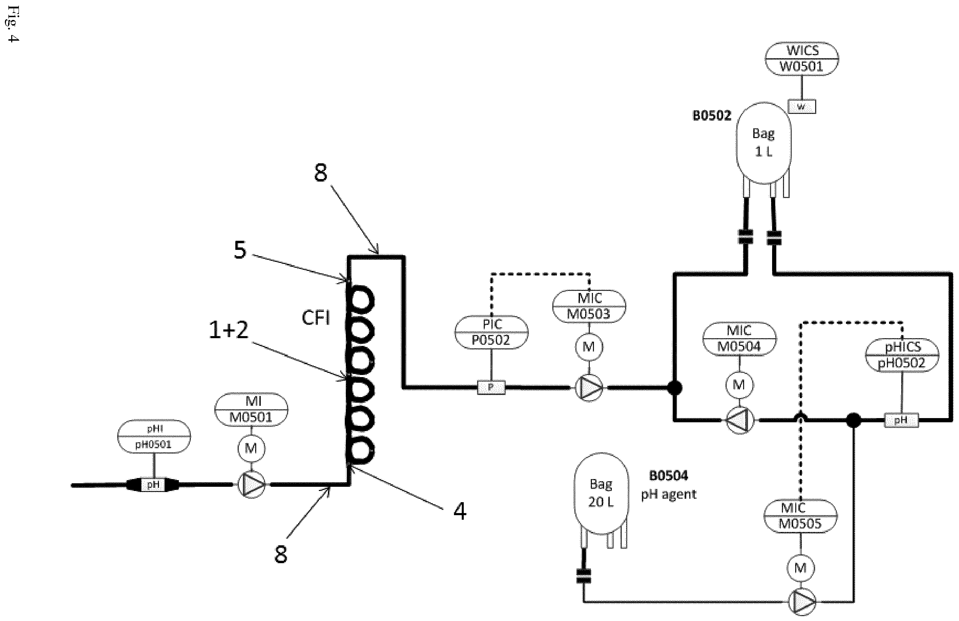

**Fig. 4** zeigt ein Fließbild der Virusinaktivierung mit anschließender Anpassung des pH-Wertes, wobei das die Vorrichtung zur Virusinaktivierung lediglich schematisch dargestellt ist.
**Fig. 5** zeigt einen eckigen Rahmen zum Wickeln des Reaktorrohrs
**Fig. 6** zeigt mehrere Rahmen montiert auf einem Gestell.

### Beispiel 1:

Für die experimentellen Untersuchungen wurde ein Schlauchinnendurchmesser von 1,6 mm gewählt. Der Rohreaktor wurde auf Rahmen mit folgenden Dimensionen -

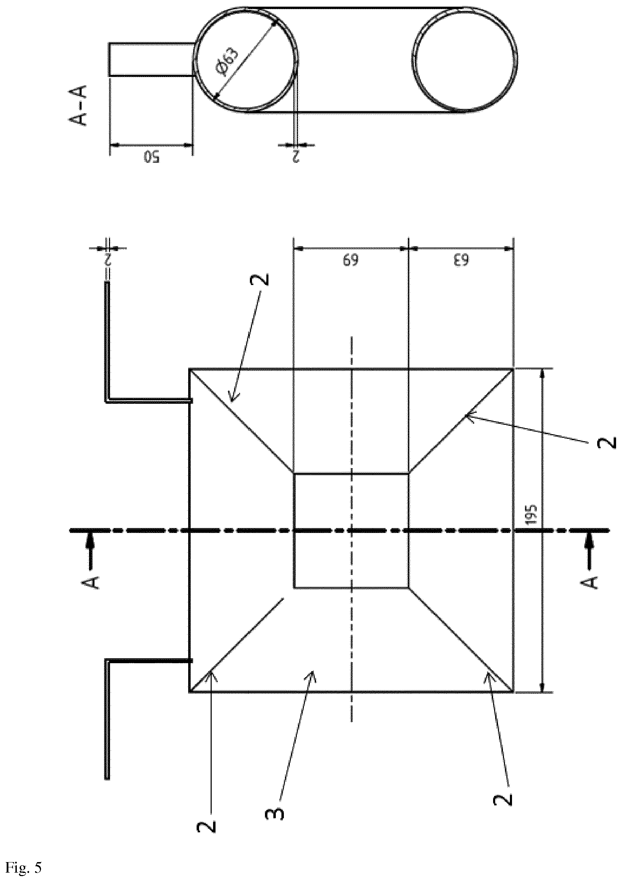

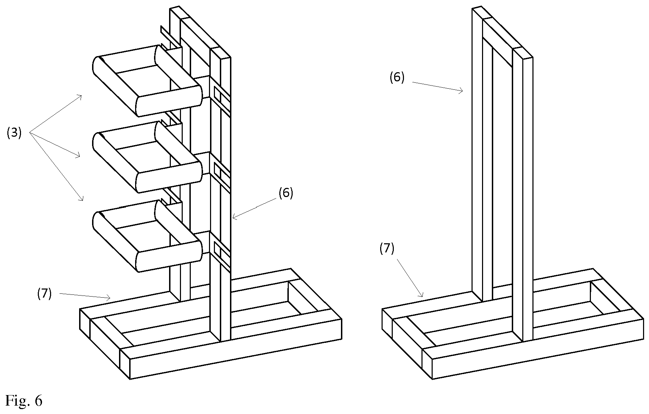

Rahmendurchmesser von 63 mm; äußere Kantenlänge der Rahmen 195 mm - gewickelt. Die Rahmen wurden entsprechend nach **Fig. 5** gefertigt und auf einem Gestell nach **Fig. 6** montiert. Pro Arm wurden stets 11 Wicklungen mit minimalem Abstand ausgeführt. Die pro Rahmen verwendete Schlauchlänge verhält sich unter der Annahme konstanter Windungszahlen pro Arm proportional zum Rahmendurchmesser.

Hierbei wurde der Ausgang des oberen Rahmens mit dem Eingang des darunter liegenden Rahmens verbunden, sodass die Schlauchwicklung der Rahmen von oben nach unten durchlaufen wurde. Alternativ kann man auch von unten nach oben oder in der Horizontalen durchströmen. Die Testanlage wurde mit einem Volumenstrom von rund 3 mL/min durchströmt.

Die Versuche zur Verweilzeitmessung in der Vorrichtung zur kontinuierlichen Virusinaktivierung wurden mit Hilfe einer UV-Messung am Austritt des Systems durchgeführt.

Als Tracer-Substanz wurde eine Vitamin B12 Lösung mit einer Konzentration von 0,25 g/L verwendet, da Vitamin B12 UV-Licht bei einer Wellenlänge von 280 nm absorbiert und sich somit als Indikator eignet.

Zunächst wurde die Vorrichtung mit destilliertem Wasser gespült. Zum Zeitpunkt k wurde am Eintritt der Virusinaktivierung auf die Tracer-Lösung umgeschaltet und die Aufzeichnung des Messsignals des UV-Sensors gestartet (Es wurde folglich eine Stufenfunktion der Tracer-Lösung auf das System aufgegeben. Wenn das UV-Signal am Austritt des Systems dem UV-Signal der Tracer-Lösung entsprach, konnten die Versuche beendet werden, da das System ab diesem Zeitpunkt komplett mit Tracer-Lösung gefüllt und somit die Antwort des Systems auf die Stufenfunktion vollständig aufgezeichnet war.

**Die Arbeiten, die zu dieser Anmeldung geführt haben, wurden gemäß der Finanzhilfevereinbarung "Bio.NRW: MoBiDiK** - **Modulare Bioproduktion** - **Disposable und Kontinuierlich" im Rahmen des Europäischen Fonds für regionale Entwicklung (EFRE) gefördert.**

## Patentansprüche

1. Verfahren zur kontinuierlichen Virusinaktivierung eines zu inaktivierenden Produktstroms in einem Reaktor (1) mit einem hydraulischen Durchmesser von 0,01 mm bis 6 mm, bevorzugt 0,5 mm bis 3 mm, umfassend folgende Schritte:
a. Bereitstellen des zu inaktivierenden Produktstroms,
b. Einstellung der vireninaktivierenden Bedingungen, durch Einstellung eines pHs auf einen Wert ≤ 4, von Detergenzien, einer UV- oder thermischen Behandlung,
c. Einbringen in den Produktstrom eines, mit dem Produktstrom nicht mischbaren, Trennmediums zur Segmentierung des Produktstroms,
d. Zufuhr und Durchlaufen des segmentierten Produktstroms aus c) unter vireninaktivierenden Bedingungen in einer Verweilstrecke gebildet durch den Reaktors (1)
e. Ausströmen aus der Verweilstrecke.

2. Verfahren nach Anspruch 1, wobei im Schritt b) der pH-Wert des Produktstroms auf einen Wert ≤ 4 eingestellt wird, sofern der pH-Wert des zu inaktivierenden Guts nicht bereits den geforderten Wert hat.

3. Verfahren nach einem der Ansprüche 1 oder 2, **dadurch gekennzeichnet, dass** die zu inaktivierende Lösung eine Lösung von Makromolekülen, bevorzugt eine Protein- oder Peptidlösung, besonders bevorzugt eine Antikörperlösung ist.

4. Verfahren nach einem der Ansprüche 1 bis 3, ferner umfassend einen Schritt f) wobei im Schritt f) eine kontinuierliche Abtrennung des Trennmediums erfolgt.

## Claims

1. Method for continuous virus inactivation of a product stream that is to be inactivated in a reactor (1) having a hydraulic diameter of 0.01 mm to 6 mm, preferably 0.5 mm to 3 mm, comprising the following steps:
a. Provision of the product stream that is to be inactivated,
b. Setting the virus-inactivating conditions, by setting a pH to a value ≤ 4, by detergents, a UV or thermal treatment,
c. Introducing a separating medium that is immiscible with the product stream into the product stream to segment it,
d. Feed and passage through of the segmented product stream from c) under virus-inactivating conditions in a detention segment formed by the reactor (1),
e. Outflow from the detention segment.

2. Method according to Claim 1, wherein, in step b), the pH of the product stream is set to ≤ 4, provided that the pH of the material that is to be inactivated does not already have the required pH.

3. Method according to either of Claims 1 and 2, **characterized in that** the solution that is to be inactivated is a solution of macromolecules, preferably a protein or peptide solution, particularly preferably an antibody solution.

4. Method according to any one of Claims 1 to 3, further comprising a step f), wherein, in step f), the separating medium is separated off continuously.

## Revendications

1. Procédé d'inactivation en continu de virus d'un flux de produit à inactiver dans un réacteur (1) comprenant un diamètre hydraulique de 0,01 mm à 6 mm, de préférence de 0,5 mm à 3 mm, comprenant les étapes suivantes :
a. fourniture du flux de produit à inactiver,
b. ajustement des conditions d'inactivation de virus par ajustement d'un pH à une valeur ≤ 4, par des détergents, un traitement aux UV ou un traitement thermique,
c. introduction dans le flux de produit d'un milieu de séparation non miscible avec le flux de produit, pour la segmentation du flux de produit,
d. alimentation et passage du flux de produit segmenté de c) dans des conditions d'inactivation de virus dans une section de séjour formée par le réacteur (1),
e. évacuation hors de la section de séjour.

2. Procédé selon la revendication 1, dans lequel, à l'étape b), la valeur pH du flux de produit est ajustée à une valeur ≤ 4, dans la mesure où la valeur pH du produit à inactiver ne possède pas déjà la valeur requise.

3. Procédé selon l'une quelconque des revendications 1 et 2, **caractérisé en ce que** la solution à inactiver est une solution de macromolécules, de préférence une solution de protéines ou de peptides, en particulier de préférence une solution d'anticorps.

4. Procédé selon l'une quelconque des revendications 1 à 3, comprenant en outre une étape f), une séparation en continu du milieu de séparation se produisant à l'étape f).
